# EUROPEAN PATENT APPLICATION

(11) **EP 1 275 646 A1**
(43) Date of publication of application: **15.01.2003**
(21) Application number: 01917558.7
(22) Date of filing: 28.03.2001
(51) Int. Cl.: C07D 403/04, A61K 31/404, A61P 43/00, A61P 25/28, A61P 21/00, A61P 25/16, A61P 25/14, A61P 27/02, A61P 27/06, A61P 25/00

(54) **INDOLYLPYRROLE DERIVATIVES AND CELL DEATH INHIBITORS**

(30) Priority: 30.03.2000 JP 2000094908
(71) Applicant: SAGAMI CHEMICAL RESEARCH CENTER, Ayase-shi, Kanagawa 252-1193 (JP); Asakai, Rei, Saitama-shi, Saitama 330-0801 (JP)
(72) Inventor: ASAKAI, Rei, Saitama-shi, Saitama 330-0801 (JP); SODEOKA, Mikiko, Sendai-shi, Miyagi 980-8577 (JP); KATOH, Miho, Ebina-shi, Kanagawa 243-0414 (JP)
(74) Representative: Albrecht, Thomas, Dr.
(86) International application number: JP0102584
(87) International publication number: WO01074807

(57) **Abstract**

The invention provides a compound represented by the following formula (I) useful for inhibiting death of cells, the drug being expected as a preventive or a remedy for the progress of various diseases wherein cell death participates in progress and exacerbation thereof: 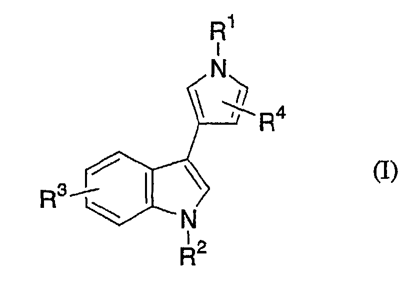 and, a cell death inhibitor, a drug or a preservative for cells, organs or tissues or cells, each comprising the derivative as an active ingredient.

## Description

### Technical Field

The present invention relates to a cell death inhibitor capable of inhibiting cell death induced by various substances in living body or foreign stimulants, or stimuli such as temperature, radiation and so on; its use as drugs for treating neurodegenerative diseases, diseases of circulatory organs, hepatitis, renal diseases, inflammatory skin disorders, radiation disorders, viral diseases, prion diseases, functional deficiency of transplanted organs, or the like, or preventing progress of the symptoms of the diseases; use as preservatives for organs, tissues and cells isolated from a living body.

### Background of the Invention

Recent progress of the study as to cell death have revealed that cell death of cells essential for living body, particularly apoptosis is involved in progress and exacerbation of a variety of diseases (*Science*, Vol. 267, p. 1456, 1995). Apoptosis is a type of cell death in which cells commit a death using their own molecular machinery, characterized generally by (1) chromatin aggregation, (2) cell shrinkage, (3) blebbing of plasma membrane (formation of processes), (4) nuclear fragmentation, (5) formation of apoptotic bodies, (6) DNA fragmentation, and (7) phagocytosis (scavenging cell debris) by neighboring cells and macrophages. In contrast, there is another type of cell death, called necrosis, characterized by cell swelling and lysis, which occurs without executing the apoptotic processes when cells are exposed to excessive radiation, heat, noxious stimulants or the like. However, the cell death caused by the own molecular machinery does not always show a full set of the apoptosis characteristics described above, depending on species of cells, environments under which cells are present, and species and strength of cell death stimulants. Likewise, necrosis in view of pathology sometimes contains a cell death which some own molecular machinery is responsible for. In the invention, such cell death is also included in apoptosis.

Examples of the diseases whose progress and exacerbation are caused by apoptotic cell death are as follows: neurodegenerative diseases such as Alzheimer's disease [*Bio Science terminology library: apoptosis*/*separate volume of Jikken Igaku (Experimental Medicine),* p. 168, 1996], spinal muscular atrophy (SMA) [*Bio Science terminology library: apoptosis*/*separate volume of Jikken Igaku (Experimental Medicine),* p. 173, 1996], amyotrophic lateral screrosis (ALS) [*Bio Science terminology library: apoptosis*/*separate volume of Jikken* *Igaku (Experimental Medicine),* p. 176, 1996], Parkinson's disease (*J. Neurochem*., Vol. 69, p. 1612, 1997), Huntington's disease (*J*. *Neurosci.,* Vol. 15, p. 3775, 1995), pigmentary degeneration of the retina and glaucoma [*Bio Science terminology library: apoptosis*/*separate volume of Jikken Igaku (Experimental Medicine),* p. 196, 1996], cerebellar degeneration and neonatal jaundice (*Progress in Drug* Research, Vol. 48, p. 55, 1997); myasthenia gravis (*J*. *Clinical Investigation,* Vol. 99, p. 2745, 1997); brain ischemia from apoplexy and the like, and successive delayed neuronal death (DND) [*Bio Science terminology library: apoptosis*/*separate volume of Jikken Igaku (Experimental Medicine),* p. 180, p. 182, 1996], ischemic heart disease due to myocardial infarction (myocardial ischemia and disorder after reperfusion), viral myocarditis, autoimmune myocarditis (congestive cardiomyopathy and chronic myocarditis), myocardial disorders or death due to hypertrophic heart and heart failure, arrythmogenic right ventricular cardiomyopathy [*Bio Science terminology library: apoptosis*/*separate volume of Jikken Igaku (Experimental Medicine),* p. 198, 1996; *Kekkan to Naihi (Blood Vessel and Endothelium),* Vol. 7, p. 357, p. 364, p. 370, 1997]; alcoholic hepatitis, viral hepatitis [*Bio Science terminology library: apoptosis*/*separate volume of Jikken Igaku (Experimental Medicine)*, p. 190, 1996], renal diseases such as glomerulonephritis, hemolytic uremic syndrome and the like [*Bio Science terminology library: apoptosis*/*separate volume of Jikken Igaku (Experimental Medicine),* p. 192, 1996], acquired immunodeficiency syndrome (AIDS) [*Bio Science terminology library: apoptosis*/*separate volume of Jikken Igaku (Experimental Medicine),* p. 156, 1996; *Ketsueki, Meneki, Shuyou (Blood, Immunity, Cancer),* Vol. 2, p. 432, 1997], inflammatory skin disorders such as toxic epidermal necrolysis (TEN) and multiform exudative erythema, alopecia, graft versus host disease (GVH) [*Bio Science terminology library: apoptosis*/*separate volume of Jikken Igaku (Experimental Medicine),* p. 194, 1996], radiation disorders [*Bio Science terminology library: apoptosis*/*separate volume of Jikken Igaku (Experimental Medicine),* p. 160, 1996], side effects due to anti-cancer drugs, anti-viral drugs and the like, disorders due to toxic agents such as sodium azide, potassium cyanide and the like [*Bio Science terminology library: apoptosis*/*separate volume of Jikken Igaku (Experimental Medicine),* p. 162, 1996], sepsis *(Critical Care Medicine,* Vol. 25, p. 1298, 1996), osteomyelo-dysplasia such as aplastic anemia and the like *(Leukemia,* Vol. 7, p. 144, 1993), insulin dependent diabetes (*Diabetes,* Vol. 44, p. 733, 1995), prion diseases such as Creutzfeldt-Jakob's disease (*J. Neural Transmission, Supplementum,* Vol. 50, p. 191, 1997), and so on. In organ transplantation, it has been suggested that apoptosis due to reactive oxygen species and various chemical mediators generated after reperfusion of anoxic organs by isolation or cardiac arrest of a donor is responsible for functional deficiency of transplanted organs (for example, *Ishoku (Transplantation),* Vol. 27, p. 15, 1992). Probably, rejection reaction after transplantation of an organ, tissues, or cells may be a result of apoptosis of the transplanted cells, which occurs when they are attacked by recipient immune cells. It is thus reasonably concluded that chemical compounds capable of inhibiting cell death can be a promising drug that heals these diseases effectively, or inhibits or stops progress and exacerbation of the symptoms of these diseases.

In the transplantation of organs or tissues, graft survival rate after transplantation depends on the preserving conditions of the organs or tissues isolated from a donor. Accordingly, it is expected to improve organ and tissue preservation by adding chemical compounds inhibiting cell death into preservation liquids for the organs and tissues. Unlike immortalized cells or cancer cells, primary cultured cells isolated from a living body are usually difficult to culture *in vitro.* For long time cultivation, appropriate concentration of additives including various growth factors are required in the culture medium depending on species of the cells, and apoptosis easily occurs in case that the culture conditions are improper. When cells are cultured for research or medical purposes, it is expected that addition of a chemical compound inhibiting cell death would lead successful cell cultivation.

Apoptosis is known to be triggered by a wide variety of physiological substances such as cytokines including interleukins, hormones including glucocorticoids, excitotoxic amino acids including glutamic acid and NMDA, and membrane proteins represented by Fas ligand, depending on cell types. It is also triggered by deprivation of a specific growth factor or the like in some cell types. There are common apoptosis triggers irrespective of cell type, such as reactive oxygen species generators including hydrogen peroxide and the like, NO generators including SNP and the like, heat, and radiation. A number of chemical compounds are also reported to be able to induce apoptosis. Recent studies have shown that apoptotic signal transduction systems where a variety of signal transduction systems participate at the upstream, appear to converge on caspase activating mechanisms at the downstream, the caspases being a series of cysteine protease (*Cell,* Vol. 91, p. 443, 1997), though their precise molecular mechanisms should be investigated in future.

Substances heretofore known as apoptosis inhibitors are, depending on species of the cells, a variety of growth factors and nutrient factors, physiological inhibitors such as hormones and the like, antioxidants such as N-acetyl-cysteine and the like, and modified peptide-type caspase inhibitors. Among them, some of peptide-type growth factors and neurotropic factors have been clinically used for the recovery of hematopoietic cells depleted after chemotherapy and for preventing cell death of neurons from neuro-degenerative diseases and trauma (*Proc. Natl. Acad. Sci. U.S.A*., Vol. 90, p. 7951, 1993; *Nature,* Vol. 367, p. 368, 1994; *Proc. Natl. Acad. Sci. U.S.A*., Vol. 89, p. 11249, 1992). The antioxidants and caspase inhibitors are only used in experiments of the cell level. Thus, it has been desired to develop an apoptosis inhibitor which is more stable *in vivo*, orally active, and a non-peptide type as well as low in molecular weight. Furthermore, since it is rare case that all apoptosis-triggering physiological factors and its inhibiting factors of the individual cells have been successfully identified in actual diseases, there is a demand for an entirely new type of cell death inhibitor which is also expected to be beneficial for the diseases where the factors are unidentified.

At present, Euro-Collins' solution and University of Wisconsin solution are generally used as organ preservation solutions for transplantation (*Ishoku (Transplantation),* Vol. 27, p. 172, 1992). Supplementation of antioxidants and radical scavengers to such preservation solutions in order to ameliorate damages of reactive oxygen has been reported to have beneficial effects on organ preservation (for example, *Ishoku (Transplantation),* Vol. 27, p. 15, 1992; Vol. 26, p. 62, 1991; Vol. 25, p. 596, 1990; Trans Proc., Vol. 17, p. 1454, 1985). However, the organ preservation is not fully sufficient, and higher graft survival rate is still desired.

On the other hand, only a limited number of synthetic examples of the indolylpyrrole derivatives have been reported (*J. Org. Chem.,* Vol. 39, p. 1980, 1974; *Ind. J. Chem. Sect. B*, Vol. 32B, p. 662, 1993; *Tetrahedron,* Vol. 53, p. 8565, 1997; *J. Org. Chem.,* Vol. 62, p. 2649, 1997; *J. Chem. Soc.,* Perkin Trans 1, p. 3087, 1998; *Tetrahedron* Lett., Vol. 35, p. 1689, 1994; WO99/28315 but there are many reports on the 3,3'-biindole derivatives in which benzene ring is fused at 4,5-position of the pyrrole); however, there has been no report that these compounds have anti-cell death effect.

### Disclosure of the Invention

The invention aims to provide a compound useful for inhibiting death of cells, the drug being expected as a preventive or a remedy for the progress of various diseases wherein cell death participates in progress and exacerbation thereof.

As a result of the extensive studies for achieving the above, the present inventors have found that the below-mentioned indolylpyrrole derivatives exhibit a cell death inhibiting action and have accomplished the invention.

Namely, the invention provides an indolylpyrrole derivative represented by the following formula (I): 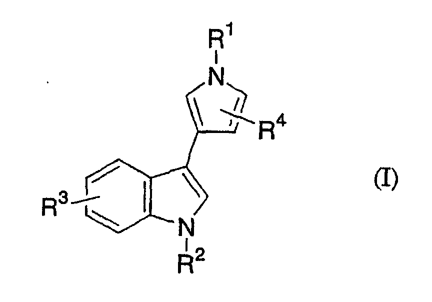 wherein R¹ and R² each independently represents hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or aryloxycarbonyl group which may possess substituent(s), an alkyl- or arylthiocarbonyl group which may possess substituent(s), an aminocarbonyl group which may possess substituent(s), an aminocarbonyloxy group which may possess substituent(s), an alkyl- or arylsulfonyl group which may possess substituent(s), an alkoxyl group which may possess substituent(s), an aryloxy group which may possess substituent(s), or hydroxyl group; R³ represents substituent(s) on an indole ring (at 2-, 4-, 5-, 6-, 7- or more than one position(s) as position number of the indole ring, in the last case substituents may be the same or different), hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or aryloxycarbonyl group which may possess substituent(s), an alkoxy- or aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group which may possess substituent(s), an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group, oxysulfonyl group, cyano group, nitro group, an amino group which may possess substituent(s), or a halogen atom; R¹ and R³, R¹ and R⁴, R² and R⁴, R³ and R⁴, R² and R³, two R³, or two R⁴ (but except the benzene ring fused at 4,5-positions of the pyrrole ring) may be combined to form a hydrocarbon chain or a hydrocarbon chain containing heteroatom(s) which may possess substituent(s); R⁴ represents substituent(s) on a pyrrole ring (at 2-, 4-, 5-, or more than one position(s) as position numbering of pyrrole ring, in the last case substituents may be the same or different), hydrogen atom (but except the case of R¹=R³=H, R²=CH₂Ph), an alkyl group which may possess substituent(s), an alkenyl group which may possess substituent(s), an alkynyl group which may possess substituent(s), an aryl group which may possess substituent(s) (but except the 3-indolyl group substituted at 4-position which may possess substituent(s)), an acyl group which may possess substituent(s) (but except the benzoyl group at 4-position in case of R¹=R³=H, R²=Et, and 4-methylbenzoyl group at 4-position in case of R¹=1H-imidazol-4-ylmethyl, R²=Me, R³=H) an acyloxy group which may possess substituent(s), an alkyl- or aryloxycarbonyl group which may possess substituent(s) (but except the alkoxycarbonyl group at 4-position), an alkyl- or aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group, an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group (but except the monosubstituted derivative at 4-position), cyano group, nitro group, an amino group which may possess substituent(s), or a halogen atom.

The invention also provides a cell death inhibitor comprising, as an active ingredient, an indolylpyrrole derivative represented by the following formula (II): 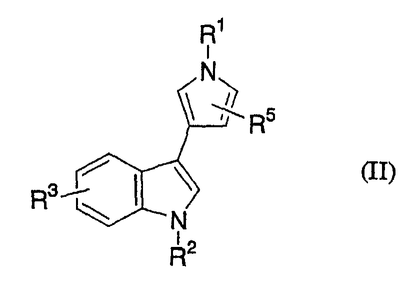 wherein R¹ and R² each independently represents hydrogen atom, an alkyl group which may possess substituent(s), an alkenyl group which may possess substituent(s), an alkynyl group, which may possess substituent(s), an aryl group which may possess substituent(s), an acyl group which may possess substituent(s), an acyloxy group which may possess substituent(s), an alkoxy- or aryloxycarbonyl group which may possess substituent(s), an alkyl- or arylthiocarbonyl group which may possess substituent(s), an aminocarbonyl group which may possess substituent(s), an aminocarbonyloxy group which may possess substituent(s), an alkyl- or arylsulfonyl group which may possess substituent(s), an alkoxyl group which may possess substituent(s), an aryloxy group which may possess substituent(s), or hydroxyl group; R³ represents substituent(s) on an indole ring, and represents, number and position (at 2-, 4-, 5-, 6-, 7- or more than one position(s) as position number of the indole ring, in the last case substituents may be the same or different), hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or aryloxycarbonyl group which may possess substituent(s), an alkoxy- or aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group which may possess substituent(s), an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group, oxysulfonyl group, cyano group, nitro group, an amino group which may possess substituent(s), or a halogen atom; R¹ and R³, R¹ and R⁵, R² and R⁵, R³ and R⁵, R² and R³, two R³, or two R⁵ may be combined to form a hydrocarbon chain or a hydrocarbon chain containing heteroatom(s) which may possess substituent(s); R⁵ represents substituent(s) on a pyrrole ring (at 2-, 4-, 5-, or more than one position(s) as position numbering of pyrrole ring, in the last case substituents may be the same or different), hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or an aryloxycarbonyl group which may possess substituent(s), an alkoxy- or an aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group, an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group, cyano group, nitro group, an amino group which may possess substituent(s), or a halogen atom, or a pharmaceutically acceptable salt thereof; a drug for treating or preventing progress of symptoms, through inhibiting death of neurons, of neurodegenerative diseases such as Alzheimer's disease, spinal muscular atrophy (SMA), amyotrophic lateral screrosis (ALS), Parkinson's disease, Huntington's disease, pigmentary degeneration of the retina, glaucoma, or cerebellar degeneration; a drug for treating or preventing progress of symptoms, through inhibiting death of neurons, of neonatal jaundice; a drug for treating or preventing progress of symptoms, through inhibiting death of cells, of myasthenia gravis; a drug for treating or preventing progress of symptoms, through inhibiting death of neurons, of brain ischemia from apoplexy and the like, and successive delayed neuronal death (DND); a drug for treating or preventing progress of symptoms, through inhibiting death of myocardial cells, of ischemic heart disease due to myocardial infarction, viral myocarditis, autoimmune myocarditis, myocardial disorders or death due to hypertrophic heart and heart failure, or arrythmogenic right ventricular cardiomyopathy; a drug for treating or preventing progress of symptoms, through inhibiting death of hepatic cells, of alcoholic hepatitis or viral hepatitis; a drug for treating or preventing progress of symptoms, through inhibiting death of renal cells, of renal diseases such as glomerulonephritis, hemolytic uremic syndrome and the like; a drug for treating or preventing progress of symptoms, through inhibiting excessive death of T-cells, of acquired immunodeficiency syndrome (AIDS); a drug for treating or preventing progress of symptoms, through inhibiting cell death, of inflammatory skin disorders such as toxic epidermal necrolysis (TEN), multiform exudative erythema and the like, alopecia, or graft versus host disease (GVH); a drug for treating or preventing disorders or side effects, through inhibiting cell death, of radiation disorders or disorders due to toxic agents including side effects due to drugs such as anti-cancer drugs, anti-viral drugs and the like; a drug for treating or preventing progress of symptoms, through inhibiting cell death, of sepsis; a drug for treating or preventing progress of symptoms, through inhibiting death of cells derived from bone marrow, of osteomyelo-dysplasia such as aplastic anemia and the like; a drug for treating or preventing progress of symptoms, through inhibiting cell death, of insulin dependent diabetes; a drug for treating or preventing progress of symptoms, through inhibiting death of neurons, of prion diseases; a drug for treating or preventing functional deficiency of transplanted organs, tissues or cells at transplantation of organs, tissues or cells; a preservative for organs, tissues and cells.

The following will explain the invention in detail.

### Best Mode for Carrying Out the Invention

The indolylpyrrole derivatives according to the invention can be synthesized according to the methods shown in Examples.

In the present description, the alkyl group in the "an alkyl group which may possess substituent(s)" may be any of linear, branched, or cyclic one, and may be exemplified by an alkyl group having 1 to 30 carbon atoms, more concretely, methyl group, ethyl group, propyl group, isopropyl group, butyl group, isobutyl group, s-butyl group, t-butyl group, pentyl group, isopentyl group, neopentyl group, hexyl group, heptyl group, octyl group, nonyl group, decyl group, cyclopropyl group, cyclobutyl group, cyclopentyl group, cyclohexyl group, cycloheptyl group, undecyl group, dodecyl group, tridecyl group, tetradecyl group, pentadecyl group, hexadecyl group, 14-methylpentadecyl group, 6-methylpentadecyl group, octadecyl group, eicosyl group, tetracosyl group, and the like.

In the present description, the alkenyl group in the "an alkenyl group which may possess substituent(s)" may be any of linear, branched, or cyclic one, and may be exemplified by an alkenyl group having 2 to 30 carbon atoms. Concrete examples thereof include allyl group, vinyl group, crotyl group, 1-penten-1-yl group, 2-penten-1-yl group, 3-penten-1-yl group, 1-hexen-1-yl group, 2-hexen-1-yl group, 3-hexen-1-yl group, 2-cyclohexenyl group, 2-cyclopentenyl group, 8-heptadecen-1-yl group, 8,11-heptadecadien-1-yl group, 8,11,14-heptadecatrien-1-yl group, 4,7,10,13-nonadecatetraen-1-yl group, 9-octadecen-1-yl group, 9,12-octadecadien-1-yl group, 9,12,15-octadecatrien-1-yl group, 6,9,12-octadecatrien-1-yl group, 5,8,11,14-eicosatetraen-1-yl group, 5,8,11,14,17-eicosapentaen-1-yl group, and 4,7,10,13,16,19-docosahexane-1-yl group.

In the present description, the alkynyl group in the "an alkynyl group which may possess substituent(s)" may be any of linear, branched, or cyclic one, and may be exemplified by an alkynyl group having 2 to 30 carbon atoms. Concrete examples thereof include ethynyl group, propargyl group, 1-pentyn-1-yl group, 2-pentyn-1-yl group, 3-pentyn-1-yl group, 1-octyn-1-yl group, and 8-heptadecyn-1-yl group.

In the present description, the aryl group in the "an aryl group which may posses substituent(s)" includes a heteroaryl group, and may be exemplified by phenyl group, naphthyl group, anthranyl group, pyrenyl group, biphenyl group, 4-pyridyl group, 2-pyridyl group, pyrimidinyl group, pyrazinyl group, piperazinyl group, pyrazolyl group, imidazolyl group, quinolyl group, pyrrolyl group, indolyl group, furyl group and the like.

In the present description, the acyl group in the "an acyl group which may possess substituent(s)" or "an acyloxy group which may possess substituent(s)" may be any of linear, branched, cyclic, saturated, unsaturated, aliphatic or aromatic one, and may be exemplified by an acyl group having 2 to 30 carbon atoms, more concretely, acetyl group, propionyl group, isopropionyl group, pivaloyl group, oleoyl group, cyclohexylcarbonyl group, acryloyl group, crotonoyl group, benzoyl group, naphthoyl group, nicotinoyl group, and the like.

In the present description, the alkoxy- or aryloxycarbonyl group in the "an alkoxy- or aryloxycarbonyl which may possess substituent(s)" or "an alkoxy- or aryloxycarbonyloxy which may possess substituent(s)" may be any of linear, branched, cyclic, saturated, unsaturated, aliphatic or aromatic one. The examples include methoxycarbonyl group, ethoxycarbonyl group, propyloxycarbonyl group, isopropyloxycarbonyl group, butoxycarbonyl group, s-butoxycarbonyl group, t-butoxycarbonyl group, cyclopentyloxycarbonyl group, cyclohexyloxycarbonyl group, benzyloxycarbonyl group, allyloxycarbonyl group, phenyloxycarbonyl group, pyridyloxycarbonyl group, and the like.

In the present description, the alkyl- or arylthiocarbonyl group in the "an alkyl- or arylthiocarbonyl which may possess substituent(s)" or "an alkyl- or arylthiocarbonyloxy which may possess substituent(s) "may be any of linear, branched, cyclic, saturated, unsaturated, aliphatic or aromatic one. The examples include methylthiocarbonyl group, ethylthiocarbonyl group, propylthiocarbonyl group, isopropylthiocarbonyl group, butylthiocarbonyl group, t-butylthiocarbonyl group, cyclopentylthiocarbonyl group, cyclohexylthiocarbonyl group, benzylthiocarbonyl group, phenylthiocarbonyl group, pyridylthiocarbonyl group, and the like.

In the present description, the aminocarbonyl group which may possess substituent(s) in the "an aminocarbonyl which may possess substituent(s)" or "an aminocarbonyloxy which may possess substituent(s)" may be an unsubstituted carbamoyl group, or a carbamoyl group which is substituted by alkyl group(s) which may possess substituent(s), aromatic group(s) which may possess substituent(s), hydroxyl group, alkoxyl group(s) which may possess substituent(s), amino group(s) which may possess substituent(s), and the like. The examples include carbamoyl group, ethylaminocarbonyl group, propylaminocarbonyl group, isopropylaminocarbonyl group, butylaminocarbonyl group, t-butylaminocarbonyl group, cyclopentylaminocarbonyl group, cyclohexylaminocarbonyl group, benzylaminocarbonyl group, phenylaminocarbonyl group, pyridylaminocarbonyl group, and the like.

In the present description, the alkyl- or arylsulfonyl group in the "an alkyl or arylsulfonyl which may possess substituent(s)" may be any of linear, branched, cyclic, saturated, unsaturated, aliphatic or aromatic one. The examples include methanesulfonyl group, ethanesulfonyl group, benzenesulfonyl group, cyclohexanesulfonyl group, naphthalenesulfonyl group, and the like.

In the present description, the alkyl- or arylsulfinyl group in the "an alkyl- or an arylsulfinyl which may possess substituent(s)" may be any of linear, branched, cyclic, saturated, unsaturated, aliphatic or aromatic one. The examples include methanesulfinyl group, ethanesulfinyl group, benzenesulfinyl group, cyclohexanesulfinyl group, naphthalenesulfinyl group, and the like.

In the present description, the alkoxyl group or aryloxy group in the "an alkoxyl group or an aryloxy group which may possess substituent(s)" may be any of linear, branched, cyclic, saturated, unsaturated, aliphatic or aromatic one, and may be exemplified by an alkoxy group or an aryloxy group having 2 to 30 carbon atoms. The particular examples include methoxy group, ethoxy group, propyloxy group, t-butoxy group, allyloxy group, cyclopentyloxy group, cyclohexyloxy group, benzyloxy group, phenoxy group, and the like.

In the present description, the alkyl- or arylthio group in the "an alkyl- or arylthio which may possess substituent(s)" may be any of linear, branched, cyclic, saturated, unsaturated, aliphatic or aromatic one, and may be exemplified by an alkyl- or arylthio group having 2 to 30 carbon atoms. The particular examples include methylthio group, ethylthio group, propylthio group, t-butylthio group, allylthio group, cyclopentylthio group, cyclohexylthio group, benzylthio group, phenylthio group, and the like.

In the present description, the "an amino group which may possess substituent(s)" may be an unsubstituted amino group, or an amino group which is substituted by alkyl group(s), aromatic group(s), acyl group(s), sulfonyl group(s) and the like. The examples include ethylamino group, propylamino group, isopropylamino group, butylamino group, t-butylamino group, benzylamino group, phenylamino group, pyridylamino group, piperazinyl group, indolinyl group, acetylamino group, benzoylamino group, benzenesulfonyl group, methanesulfonyl group and the like.

In the present description, "a halogen atom" may be fluorine atom, chlorine atom, bromine atom, and iodine atom.

The examples of substituents which may be present in the above-mentioned alkyl group, alkenyl group, alkynyl group, aryl group, acyl group, acyloxy group, alkoxy- or aryloxycarbonyl group, alkoxy- or aryloxycarbonyloxy group, alkylthio- or arylthiocarboyl group, alkylthio- or arylthiocarboyloxy group, aminocarbonyl group, aminocarbonyloxy group, alkoxyl group or aryloxy group, alkyl- or arylthio group, alkyl or arylsulfonyl group, alkyl or arylsulfinyl group, amino group, and the like include alkyl groups, alkenyl groups, alkynyl groups, aryl groups, acyl groups, acyloxy group, alkoxy- or aryloxycarbonyl group, alkoxy or aryloxycarbonyloxy group, alkylthio- or arylthiocarbonyl group, alkylthio- or arylthiocarboyloxy group, aminocarbonyl group, aminocarbonyloxy group alkoxyl group, aryloxy group, alkyl- or arylthio group, alkyl- or arylsulfonyl group, alkyl- or arylsulfinyl group, and particular examples thereof are the same as mentioned above. The other substituents may be exemplified by halogen groups, nitro group, amino groups (which may possess substituent(s) such as acyl group(s), alkoxy- or aryloxycarbonyl group(s), carbamoyl group(s), substituted sulfonyl group(s), alkyl group(s), cycloalkyl group(s), aryl group(s), and the like), cyano group, hydroxyl group, carboxyl group, oxysulfonyl group, epoxy group and the like, as well as aralkyl groups such as benzyl group, phenethyl group, naphthylmethyl group, and the like.

As examples of two R³ groups or two R⁵ groups combined to form a hydrocarbon chain or a hydrocarbon chain containing heteroatom(s) which may possess substituent(s), rings fused to the indole ring or pyrrole ring are represented. The fused ring may be exemplified by saturated or unsaturated aliphatic ring such as cyclopentane ring, cyclohexane ring, cyclohexene ring and the like; saturated or unsaturated heterocyclic ring such as pyrrolidine ring, tetrahydrofuran ring, imidazolidine ring, imidazoline ring, piperidine ring, morpholine ring, tetrahydrothiophene ring, and the like; aromatic ring such as benzene ring, naphthalene ring, indane ring, acenaphthene ring, fluorene ring, phenanthrene ring, and the like; heteroaromatic ring such as furan ring, thiophene ring, pyrrole ring, imidazole ring, pyridine ring, pyrimidine ring, benzofuran ring, indole ring, quinoline ring, and the like.

As to the pharmaceutically acceptable salts, the compound having an acid part may formed a salt with an inorganic base or organic base, for example, an alkaline metal salt such as sodium salt, potassium salt, or the like; an alkaline earth metal salt such as calcium salt, magnesium salt, or the like; ammonium salt; an aliphatic or heteroaromatic amine salt such as triethylamine salt, ethanolamine salt, lysine salt, arginine salt, quinoline salt, or the like; a quaternary ammonium salt such as tetramethylammonium or the like. The compound having a basic part may form a salt with an inorganic or organic acid, for example, hydrochloride, bromate, iodate, sulfate, nitrate, phosphate, citrate, tartrate, malate, lactate, salicylate, malonate, fumarate, succiniate, oxalate, ascorbate, or the like.

The compound according to the invention may be applied as medicament in any form selected from various forms, for example, formulations for oral administration such as tablets, capsules, powders, granules, or liquids; and formulations for parenteral administration such as injections, rectal suppositories, formulations for external use on skin, inhalant, and the like.

Solid formulations can be prepared in a form of tablets, capsules, granules, or powders by themselves, or can be prepared with using suitable additive(s). Examples of such additives include sugars such as lactose or glucose; starches; fatty acids such as stearic acid; inorganic salts such as magnesium metasilicate aluminate or anhydrous calcium phosphate; synthetic polymers such as polyvinylpyrrolidone or polyalkylene glycol; fatty acid salts such as calcium stearate or magnesium stearate; alcohols such as stearyl alcohol or benzyl alcohol; synthetic cellulose derivatives such as methyl cellulose, ethyl cellulose, carboxymethyl cellulose, or hydroxypropyl cellulose; other conventional additives such as water, gelatin, talc, vegetable oils, acacia, or the like.

Liquid formulations are prepared in a form of suspensions, syrups, or injections by using suitable additive(s) conventionally used in liquid formulations such as water, alcohols, vegetable-derived oils including soybean oil, peanut oil, sesame oil, *etc.*

Especially, examples of suitable solvents for injections include distilled water for injection, aqueous lidocain hydrochloride solution, physiological saline, aqueous glucose solution, ethanol, liquids for intravenous injection such as aqueous solutions of citric acid and sodium citrate, electrolyte solution, and the like, or mixtures thereof. These injections may be a form of predissolved one, and also a form for dissolving before use, which is composed of powder itself or powder with suitable additive(s).

The rectal suppositories may be prepared either by melting an active ingredient and base material(s) such as cacao butter, tri-, di- and monoglyceride of a fatty acid, polyethylene glycol, and the like under heating; charging the melt into a mold; and then cooling it; or by dissolving an active ingredient into polyethylene glycol, soybean oil, or the like and then covering it with gelatin film.

In the preparation of formulations for external use on skin, an active ingredient is added to vaseline, bees wax, liquid paraffin, polyethylene glycol, *etc.*, followed by either kneading it, if necessary under heating, to form ointments, or kneading it with an adhesive such as rosin, a polymer of alkyl acrylate, *etc.* and spreading the kneaded one on unwoven cloth such as polyethylene or the like to form tapes.

In the preparation of inhalant, an active ingredient is dissolved or dispersed into a propellant such as fron gas, and then a pressure container is filled up to form aerosols.

Preferred dosage of the compound according to the invention varies depending on kinds of the compositions blended, dosing times and diseases to be treated, and also ages, body weights, and symptoms of patients, but may be ordinarily in an amount of about 1-1000 mg a day, preferably 5 to 500 mg, and they may be administered in one or several dosage units per day.

The organs relating to the invention may be all organs, such as heart, lung, liver, kidney, pancreas, and intestine.

The tissues relating to the invention may be all tissues, such as skin, cornea, bone marrow, vascular systems, and bone.

In the invention, cells expected to have effects on maintenance of the cell function by transplantation or the preservation may be all types of cells (normal various cells, immortalized cell lines, cancer cells, and cells that are modified by genetic recombination techniques for disease treatment and research purposes), such as vascular cells, islet cells of Langerhans, epidermal cells, neuronal cell, and embryonic stem cells.

As far as an administration method is concerned, when the chemical compounds according to the invention are used for the preservation of organs, tissues, and cells, various routes can be selected. For example, the present compound or a pharmaceutically acceptable salt thereof can be added to a culture medium or preservation solution containing appropriate salts and nutrients. In case of organ transplantation, it can be also administered intravenously or at perfusion to a donor prior to the organ isolation.

The invention will be explained in detail in the following sections by way of Examples but the invention is, needless to say, not limited to the Examples.

### Examples

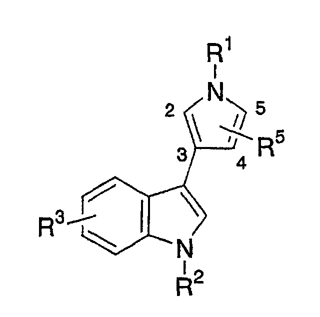

- Compound 1: R¹=CH₃, R²=R³=R⁵=H
- Compound 2: R¹=CH₃, R²=H, R³=5-CH₃, R⁵=H
- Compound 3: R¹=H, R²=CH₃, R³=H, R⁵=4-CN
- Compound 4: R¹=R²=CH₃, R³=H, R⁵=4-CN
- Compound 5: R¹=H, R²=CH₃, R³=H, R⁵=4-COOEt
- Compound 6: R¹=R²=CH₃, R³=H, R⁵=4-COOEt
- Compound 7: R¹=R²=CH₃, R³=H, R⁵=4-COOH
- Compound 8: R¹=R²=CH₃, R³=H, R⁵=4-CONH(CH₂)₁₃CH₃
- Compound 9: R¹=H, R²=CH₃, R³=H, R⁵=4-CH₃
- Compound 10: R¹=R²=CH₃, R³=H, R⁵=4-CH₃
- Compound 11: R¹=R²=CH₃, R³=H, R⁵=4-Ph

### Example 1

To a solution of 3-(1H-indol-3-yl)-1-methyl-2,5-dioxopyrrolidine (300 mg, 1.32 mmol) synthesized according to a known method (*Synthesis,* p.443, 1997) in THF (5 mL) was added 0.95M diisobutylaluminum hydride (5.7 mL, 5.26 mmol) dropwise slowly under ice cooling. After stirring at room temperature for 2 hours, the reaction mixture was added with saturated aqueous ammonium chloride solution and then stirred for further 30 minutes. Insoluble material was removed by filtration through celite, and the filtrate was concentrated under reduced pressure. The residue was purified by column chromatography over silica gel (ethyl acetate:n-hexane = 1:2) to obtain Compound 1 (156 mg, 60.2%) as pale brown solids.
mp 102-106°C
¹H-NMR (CDCl₃) δ 3.72 (s, 3H), 6.42 (dd, J=2.0, 2.0 Hz, 1H), 6.67 (dd, J=2.0, 2.0 Hz, 1H), 6.93 (dd, J=2.0, 2.0 Hz, 1H), 7.14 (t, J=7.9Hz, 1H), 7.20 (t, J=7.9Hz, 1H), 7.24 (d, J=2.3Hz, 1H), 7.37 (d, J=7.9Hz, 1H), 7.87 (d, J=7.9Hz, 1H), 8.00 (br s, 1H)
IR (KBr) 3310, 2910, 1700, 1620, 1520, 1460, 1420, 1282, 1245, 1225, 1100, 775, 740 cm⁻¹
MS m/z 196 (M⁺)

### Example 2

To a solution of 1-methyl-3-(5-methyl-1H-indol-3-yl)-2,5-dioxopyrrolidine (200 mg, 0.83 mmol) synthesized according to a known method (*Synthesis*, p.443, 1997) in THF (4 mL) was added 0.95M diisobutylaluminum hydride (3.6 mL, 3.3 mmol) dropwise slowly under ice cooling. After stirring at room temperature for 2 hours, the reaction mixture was added with saturated aqueous ammonium chloride solution and then stirred for further 30 minutes. Insoluble material was removed by filtration through celite, and the filtrate was concentrated under reduced pressure. The residue was purified by column chromatography over silica gel (ethyl acetate:n-hexane = 1:2) to obtain Compound 2 (112 mg, 64.5%) as pale brown solids.
mp 143-145°C
¹H-NMR (CDCl₃) δ 2.74 (s, 3H), 3.71 (s, 3H), 6.42 (dd, J=2.0, 2.0Hz, 1H), 6.67 (dd, J=2.0, 2.0Hz, 1H), 6.92 (dd, J=2.0, 2.0Hz, 1H), 7.03 (d, J=8.0Hz, 1H), 7.18 (d, J=2.3Hz, 1H), 7.25 (d, J=8.0Hz, 1H), 7.64 (s, 1H), 7.89 (br s, 1H)
IR (KBr) 3400, 2925, 1720, 1520, 1480, 1440, 1420, 1285, 1250, 1230, 1100, 800, 770, 600, 505 cm⁻¹
MS m/z 210 (M⁺)

### Example 3

Sodium hydride (60 to 72%, oily, 90 mg) was washed with pentane and then suspended in THF (4 mL). A THF solution (2 mL) of diethylcyanomethylphosphonate (0.15 mL, 1.38 mmol) was slowly added thereto, and the whole was stirred at room temperature for 30 minutes. To this mixture was added a solution of 1-methylindol-3-carboxyaldehyde (200 mg, 1.26 mmol) in THF (4 mL), and the whole mixture was stirred for 16 hours. After addition of water, the reaction mixture was extracted with diethyl ether, and the organic layer was washed with saturated aqueous sodium chloride solution, dried with sodium sulfate, and concentrated under reduced pressure. The residue was purified by column chromatography over silica gel (ethyl acetate:n-hexane = 1:2) to obtain (E)-3-(1-methyl-1H-indol-3-yl)acrylonitrile (194 mg, 84.8%) as pale yellow solids.
mp 91-94°C
¹H-NMR (CDCl₃) δ 3.81 (s, 3H), 5.73 (d, J=16.5Hz, 1H), 7.25 (t, J=7.9Hz, 1H), 7.31 (s, 1H), 7.33 (t, J=7.9Hz, 1H), 7.36 (d, J=7.9Hz, 1H), 7.50 (d, J=16.5Hz, 1H), 7.75 (d, J=7.9Hz, 1H)
IR (KBr) 3500, 3140, 2210, 1625, 1540, 1390, 750 cm⁻¹
MS m/z 182 (M⁺)

Sodium hydride (60 to 72%, oily, 90 mg) was washed with pentane and then diethyl ether (1 mL) was added. A solution of (E)-3-(1-methyl-1H-indol-3-yl)acrylonitrile (100 mg, 0.55 mmol) and tosylmethylisocyanide (100 mg, 0.55 mmol) in DMSO/diethyl ether (1:2) (2.75 mL) was added thereto, and the mixture was stirred under reflux for 2 hours. After cooling to room temperature and addition of water, the mixture was extracted with diethyl ether. The organic layer was washed with saturated aqueous sodium chloride solution, dried with sodium sulfate, and concentrated under reduced pressure. The residue was purified by column chromatography over silica gel (ethyl acetate:n-hexane = 1:1) to obtain Compound 3 (61 mg, 53.5%) as pale yellow solids.
mp 131-135°C
¹H-NMR (CDCl₃) δ 3.84 (s, 3H), 7.14 (dd, J=2.2, 2.2Hz, 1H), 7.18 (dt, J=0.9, 7.9Hz, 1H), 7.28 (dt, J=0.9, 7.9Hz, 1H), 7.36 (dd, J=2.2, 2.2Hz, 1H), 7.37 (d, J=7.9Hz, 1H), 7.58 (s, 1H), 7.76 (d, J=7.9Hz, 1H), 8.60 (br s, 1H)
IR (KBr) 3280, 2250, 1470, 1390, 1250, 1100, 760, 620 cm⁻¹
MS m/z 221 (M⁺)

### Example 4

Compound 3 (30 mg, 0.15 mmol) was dissolved in DMF (1.5 mL), and sodium hydride (60 to 72%, oily, 8.7 mg) was added thereto under ice cooling. After stirring for 10 minutes, methyl iodide (14 µL, 0.22 mmol) was added thereto, and stirred for 2 hours under gradually warming to room temperature. To this reaction mixture was added saturated aqueous sodium chloride solution, and the mixture was extracted with ethyl acetate. The extract was concentrated under reduced pressure after drying over sodium sulfate. The residue was purified by column chromatography over silica gel (ethyl acetate:n-hexane = 1:2) to obtain Compound 4 (29 mg, 84%) as colorless solids.
mp 109-112°C
¹H-NMR (CDCl₃) δ 3.74 (s, 3H), 3.83 (s, 3H), 6.94 (d, J=2.2Hz, 1H), 7.14 (d, J=2.2Hz, 1H), 7.17 (dt, J=0.9, 7.9Hz, 1H), 7.27 (dt, J=0.9, 7.9Hz, 1H), 7.35 (d, J=7.9Hz, 1H), 7.56 (s, 1H), 7.74 (d, J=7.9Hz, 1H)
IR (KBr) 3250, 2250, 1550, 1340, 1165, 840, 740 cm⁻¹
MS m/z 235 (M⁺)

### Example 5

Sodium hydride (60 to 72%, oily, 90 mg) was washed with pentane and then suspended in THF (4 mL). A THF solution (2 mL) of ethyl diethylphosphonoacetate (0.27 mL, 1.38 mmol) was slowly added thereto, and the whole was stirred at room temperature for 30 minutes. To this mixture was added a solution of 1-methylindol-3-carboxyaldehyde (200 mg, 1.26 mmol) in THF (4 mL), and the whole mixture was stirred for 18 hours. After addition of water, the reaction mixture was extracted with diethyl ether, and the organic layer was washed with saturated aqueous sodium chloride solution, dried with sodium sulfate, and concentrated under reduced pressure. The residue was purified by column chromatography over silica gel (ethyl acetate:n-hexane = 1:2) to obtain ethyl (E)-3-(1-methyl-1H-indol-3-yl)acrylate (258 mg, 89.6%) as colorless solids.
mp 94-98°C
¹H-NMR (CDCl₃) δ 1.35 (t, J=7.1Hz, 3H), 3.81 (s, 3H), 4.27 (q, J=7.1Hz, 2H), 6.41 (d, J=15.9Hz, 1H), 7.25 (t, J=7.9Hz, 1H), 7.31 (t, J=7.9Hz, 1H), 7.34 (s, 1H), 7.35 (d, J=7.9Hz, 1H), 7.89 (d, J=15.9Hz, 1H), 7.92 (d, J=7.9Hz, 1H)
IR (KBr) 3500, 3140, 3000, 1710, 1630, 1395, 1300, 1210 ,1195 cm⁻¹
MS m/z 229 (M⁺)

Sodium hydride (60 to 72%, oily, 38 mg) was washed with pentane and then diethyl ether (1 mL) was added. A solution of ethyl (E)-3-(1-methyl-1H-indol-3-yl)acrylate (100 mg, 0.44 mmol) and tosylmethylisocyanide (170 mg, 0.87 mmol) in DMSO/diethyl ether (1:2)(2.75 mL) was added thereto, and the mixture was stirred under reflux for 2 hours. After cooling to room temperature and addition of water, the mixture was extracted with diethyl ether. The organic layer was washed with saturated aqueous sodium chloride solution, dried with sodium sulfate, and concentrated under reduced pressure. The residue was purified by column chromatography over silica gel (ethyl acetate:n-hexane = 1:1) to obtain Compound 5 (73 mg, 62%) as colorless solids.
mp 159-164°C
¹H-NMR (CDCl₃) δ 1.24 (t, J=7.1Hz, 3H), 3.82 (s, 3H), 4.22 (q, J=7.1Hz, 2H), 7.02 (dd, J=2.4, 2.4Hz, 1H), 7.11 (dt, J=0.9, 7.9Hz, 1H), 7.22 (dt, J=0.9, 7.9Hz, 1H), 7.33 (d, J=7.9Hz, 1H), 7.53 (dd, J=2.4, 2.4Hz, 1H), 7.57 (s, 1H), 7.70 (d, J=7.9Hz, 1H), 8.50 (br s, 1H)
IR (KBr) 3260, 3005, 1695, 1470, 1340, 1180, 1095, 760 cm⁻¹
MS m/z 268 (M⁺)

### Example 6

Compound 5 (50 mg, 0.19 mmol) was dissolved in DMF (2 mL), and sodium hydride (60 to 72%, oily, 15 mg) was added thereto under ice cooling. After stirring for 10 minutes, methyl iodide (17 µL, 0.28 mmol) was added thereto, and stirred for 2 hours under gradually warming to room temperature. To this reaction mixture was added saturated aqueous sodium chloride solution, and the mixture was extracted with ethyl acetate. The extract was concentrated under reduced pressure after drying over sodium sulfate. The residue was purified by column chromatography over silica gel (ethyl acetate:n-hexane = 1:2 to obtain Compound 6 (52 mg, 98.6%) as pale yellow viscous liquid.
¹H-NMR (CDCl₃) δ 1.23 (t, J=7.1Hz, 3H), 3.73 (s, 3H), 3.82 (s, 3H), 4.21 (q, J=7.1Hz 2H), 6.87 (d, J=2.4Hz, 1H), 7.11 (dt, J=0.9, 7.9Hz, 1H), 7.21 (dt, J=0.9, 7.9Hz, 1H), 7.32 (d, J=7.9Hz, 1H), 7.35 (d, J=2.4Hz, 1H), 7.59 (s, 1H), 7.71 (d, J=7.9Hz, 1H)
IR (KBr) 3400, 3000, 2955, 1720, 1545, 1485, 1340, 1270, 1250, 1195, 1120, 1090, 800, 755 cm⁻¹
MS m/z 282 (M⁺)

### Example 7

Compound 6 (34 mg, 0.12 mmol) was dissolved in MeOH (1 mL), and 30% aqueous sodium hydroxide solution (1 mL) was added thereto. The mixture was stirred under reflux for 8 hours. After removal of MeOH by the concentration under reduced pressure, the mixture was acidified with 1N aqueous hydrochloric acid solution and extracted with ethyl acetate. The organic layer was dried over sodium sulfate, and then concentrated under reduced pressure. The residue was purified by column chromatography over silica gel (ethyl acetate:n-hexane=3:1) to obtain Compound 7 (29 mg, 96%) as colorless solids.
mp 230-233°C
¹H-NMR (CDCl₃) δ 3.72 (s, 3H), 3.81 (s, 3H), 6.86 (d, J=2.4Hz, 1H), 7.12 (t, J=7.6Hz, 1H), 7.22 (t, J=7.6Hz, 1H), 7.32 (d, J=7.6Hz, 1H), 7.44 (d, J=2.4Hz, 1H), 7.52 (s, 1H), 7.72 (d, J=7.6Hz, 1H)
IR (KBr) 3500, 2950, 1680, 1660, 1540, 1460, 1280, 1250, 1200, 805, 755 cm⁻¹
MS m/z 254 (M⁺)

### Example 8

To a solution of dicyclohexylcarbodiimide (6.1 mg, 0.03 mmol) and 1-hydroxybenzotriazole (4.5 mg, 0.03 mmol) in THF (0.3 mL) was added dropwise a solution of Compound 7 (4.5 mg, 0.03 mmol) in THF (0.3 mL), and the whole mixture was stirred for 2 hours. Next, tetradecylamine (5 mg, 0.024 mmol) was added thereto, and mixture was stirred for 14 hours. After filtration of the reaction mixture, the filtrate was concentrated and the resulting residue was by column chromatography over silica gel (ethyl acetate:n-hexane = 1:1) to obtain Compound 8 (5.6 mg, 63.4%) as pale yellow solids.
mp 61-65°C
¹H-NMR (CDCl₃) δ 0.88 (t, J=6.8Hz, 3H), 0.99-1.37 (m, 24H), 3.10 (dt, J=6.8, 6.8Hz, 2H), 3.69 (s, 3H), 3.84 (s, 3H), 5.90 (br t, J=6.8Hz, 1H), 6.55 (d, J=2.5Hz, 1H), 7.08 (s, 1H), 7.13 (t, J=7.9Hz, 1H), 7.26 (t, J=7.9Hz, 1H), 7.35 (d, J=7.9Hz, 1H), 7.39 (d, J=2.5Hz, 1H), 7.54 (d, J=7.9Hz, 1H)
IR (KBr) 3370, 2950, 2875, 1645, 1530, 1480, 1290, 1110, 820 cm⁻¹
MS m/z 449 (M⁺)

### Example 9

A solution of tosylmethylisocyanide (1.2 g 6.29 mmol) in dimethoxyethane (6 mL) was added to a suspension of potassium tert-butoxide (880 mg, 7.86 mmol) in DME (3 mL) dropwise slowly at -40°C, then a solution of 1-methylindol-3-carboxyaldehyde (1 g, 6.29 mmol) in dimethoxyethane (3 mL) was added thereto, and the mixture was stirred for 3 hours. The reaction mixture was poured into 5% aqueous ammonium chloride solution, and extracted with methylene chloride. The organic layer was washed with water and saturated aqueous sodium chloride solution, dried with sodium sulfate, and concentrated under reduced pressure. The residue was recrystallized from MeOH to obtain (E)-N-[2-(1-methyl-1H-indol-3-yl)-1-tosylethenyl]formamide (1.4 g, 62.9%) as colorless solids.
mp 199-201°C
¹H-NMR (CDCl₃) δ 2.43 (s, 3H), 3.83 (s, 3H), 6.68 (d, J=11.4Hz, 1H), 7.28-7.30 (m, 6H), 7.57 (s, 1H), 7.70 (d, J=11.4Hz, 1H), 7.82 (d, J=7.8Hz, 2H), 8.16 (s, 1H)
IR (KBr) 3270, 1690, 1640, 1540, 1500, 1310, 1300, 1200, 1150, 1130, 1100, 760, 700, 600, 575, 560 cm⁻¹
MS m/z 354 (M⁺)

To a solution of (E)-N-[2-(1-methyl-1H-indol-3-yl)-1-tosylethenyl]formamide (2 g, 5.6 mmol) in dimethoxyethane (30 mL) was added triethylamine (4.3 mL, 30 mmol) and phosphorus oxychloride (0.68 mL, 7.3 mmol) at -30°C, and the mixture was stirred for 4 hours. The mixture was poured into 5% aqueous ammonium chloride solution, and extracted with methylene chloride. The organic layer was washed with water and saturated aqueous sodium chloride solution, dried with sodium sulfate, and concentrated under reduced pressure. The residue was recrystallized from MeOH to obtain (E)-1-isocyano-2-(1-methyl-1H-indol-3-yl)-1-tosylethene (1 g, 52.7%) as a yellow solids.
mp 172-176°C
¹H-NMR (CDCl₃) δ 2.45 (s, 3H), 3.89 (s, 3H), 7.31-7.42 (m, 5H), 7.83 (d, J=7.6Hz, 1H), 7.90 (d, J=8.3Hz, 2H), 8.03 (s, 3H), 8.08 (s, 1H)
IR (KBr) 3500, 2125, 1610, 1540, 1360, 1340, 1160, 1110, 755, 695, 600, 580 cm⁻¹
MS m/z 336 (M⁺)

To a solution of (E)-1-isocyano-2-(1-methyl-1H-indol-3-yl)-1-tosylethene (50 mg, 0.15 mmol) and propiophenone (40 µL, 12.98 mmol) in DME (1 mL) was added a solution of potassium tert-butoxide (52 mg, 0.46 mmol) in DME (0.5 mL) dropwise slowly. After stirring for 1 hour, water was added to the reaction mixture, and the mixture was extracted with methylene chloride. The organic layer was washed with saturated aqueous sodium chloride solution, dried with sodium sulfate, and concentrated under reduced pressure. The residue was purified by column chromatography over silica gel (ethyl acetate:n-hexane = 1:1) to obtain Compound 9 (17 mg, 54.4%) as colorless solids.
mp 85-88°C
¹H-NMR (CDCl₃) δ 2.21 (s, 3H), 3.83 (s, 3H), 6.69 (br s, 1H), 7.00 (br s, 1H), 7.08 (br s, 1H), 7.13 (t, J=8.0Hz, 1H), 7.24 (t, J=8.0Hz, 1H), 7.34 (d, J=8.0Hz, 1H), 7.74 (d, J=8.0Hz, 1H), 8.05 (br s, 1H)
IR (KBr) 3440, 2950, 1250, 1080, 780, 760, 550 cm⁻¹
MS m/z 210 (M⁺)

### Example 10

Compound 9 (20 mg, 0.095 mmol) was dissolved in DMF (0.5 mL), and sodium hydride (60 to 72%, oily, 5.7 mg) was added thereto under ice cooling. After stirring for 10 minutes, methyl iodide (8.9 µL, 0.14 mmol) was added thereto, and stirred for 2 hours under gradually warming to room temperature. To this reaction mixture was added saturated aqueous sodium chloride solution, and the mixture was extracted with ethyl acetate. The extract was concentrated under reduced pressure after drying over sodium sulfate. The residue was purified by column chromatography over silica gel (ethyl acetate:n-hexane = 1:2) to obtain Compound 10 (18.4 mg, 79.2%) as pale yellow solids.
mp 98-101°C
¹H-NMR (CDCl₃) δ 2.17 (s, 3H), 3.67 (s, 3H), 3.81 (s, 3H), 6.50 (br s, 1H), 6.80 (br s, 1H), 7.04 (s, 1H), 7.12 (t, J=8.0Hz, 1H), 7.23 (t, J=8.0Hz, 1H), 7.32 (d, J=8.0Hz, 1H), 7.74 (d, J=8.0Hz, 1H)
IR (KBr) 3500, 2960, 1520, 1470, 1440, 1340, 1160, 1100, 800, 760 cm⁻¹
MS m/z 224 (M⁺)

### Example 11

1-Methylindol-3-acetamide (50 mg, 0.27 mmol) synthesized according to a known method (*J. Org. Chem*., Vol. 63, p. 6053, 1998) and ethyl phenylglyoxylate (52 mg, 0.29 mmol) were dissolved in DMF (1 mL). A solution of potassium tert-butoxide (66 mg, 0.59 mmol) in DMF (1.5 mL) was added thereto at room temperature, and the mixture was stirred at 45°C for 4 hours. The mixture was poured into water, and extracted with ethyl acetate. The organic layer was washed with saturated aqueous sodium chloride solution, dried with sodium sulfate, and concentrated under reduced pressure. The residue was purified by column chromatography over silica gel (ethyl acetate:n-hexane=5:1) to obtain 2-(1-methyl-1H-indol-3-yl)-3-phenylmaleimide (71 mg, 88%) as yellow solids.
mp 255-257°C
¹H-NMR (CDCl₃) δ 3.89 (s, 3H), 6.29 (d, J=8.0Hz, 1H), 6.70 (t, J=8.0Hz, 1H), 7.11 (t, J=8.0Hz, 1H), 7.29-7.37 (m, 3H), 7.39 (dd, J=8.0, 1.7Hz, 2H), 7.47 (d, J=8.0Hz, 1H), 8.03 (s, 1H), 11.05 (brs, 1H)
IR (KBr) 3156, 3050, 1760, 1700, 1620, 1515, 1335, 1250 cm⁻¹
MS m/z 302 (M⁺)

2-(1-Methyl-1H-indol-3-yl)-3-phenylmaleimide (50 mg, 0.16 mmol) was dissolved in DMF (1 mL), and sodium hydride (60 to 72%, oily, 9.9 mg) was added thereto under ice cooling. After stirring for 10 minutes, methyl iodide (15 µL, 2.48 mmol) was added thereto, and stirred for 2 hours under gradually warming to room temperature. To this reaction mixture was added saturated aqueous sodium chloride solution, and the mixture was extracted with ethyl acetate. The extract was concentrated under reduced pressure after drying over sodium sulfate. The residue was purified by column chromatography over silica gel (ethyl acetate:n-hexane = 1:1) to obtain 2-(1-methyl-1H-indol-3-yl)-3-phenyl-N-methylmaleimide (49 mg, 93%) as orange solids.
mp 242-246°C
¹H-NMR (CDCl₃) δ 3.16 (s, 3H), 3.88 (s, 3H), 6.40 (d, J=8.0Hz, 1H), 6.78 (t, J=8.0Hz, 1H), 7.16 (t, J=8.0Hz, 1H), 7.27-7.36 (m, 4H), 7.51 (d, J=8.0Hz, 1H), 7.94 (s, 1H)
IR (KBr) 3150, 3050, 1760, 1695, 1625, 1520, 1375, 1250 cm⁻¹
MS m/z 316 (M⁺)

To a solution of 2-(1-methyl-1H-indol-3-yl)-3-phenyl-N-methylmaleimide (100 mg, 0.32 mmol) in DMF (15 mL) was added a small amount of 10% palladium-carbon, and the whole was stirred at room temperature for overnight under hydrogen atmosphere. The palladium-carbon was removed by filtration, and the filtrate was concentrated under reduced pressure. The residue was purified by column chromatography over silica gel (ethyl acetate:n-hexane = 1:1) to obtain 1-methyl-3-(1-methyl-1H-indol-3-yl)-2,5-dioxo-4-phenylpyrrolidine (97.3 mg, 96.7%) as pale yellow solids.
mp 179-182°C
¹H-NMR (CDCl₃) δ 3.21 (s, 3H), 3.74 (s, 3H), 4.17 (d, J=5.5Hz, 1H), 4.31 (d, J=5.5Hz, 1H), 6.97 (s, 1H), 7.06 (t, J=8.7Hz, 1H), 7.17-7.27 (m, 4H), 7.29-7.40 (m, 4H)
IR (KBr) 3490, 2955, 1790, 1710, 1440, 1390, 1300, 1120, 760, 710 cm⁻¹
MS m/z 318 (M⁺)

To a solution of 1-methyl-3-(1-methyl-1H-indol-3-yl)-2,5-dioxo-4-phenylpyrrolidine (50 mg, 0.16 mmol) in THF (2 mL) was added 0.94M diisobutylaluminum hydride (0.7 mL, 0.63 mmol) dropwise slowly under ice cooling. After stirring at room temperature for 4 hours, the reaction mixture was added with saturated aqueous ammonium chloride solution and then stirred for further 30 minutes. Insoluble material was removed by filtration through celite, and the filtrate was concentrated under reduced pressure. The residue was purified by column chromatography over silica gel (ethyl acetate:n-hexane = 1:3) to obtain Compound 11 (22.5 mg, 50%) as pale yellow solids.
mp 117-119°C
¹H-NMR (CDCl₃) δ 3.72 (s, 3H), 3.74 (s, 3H), 6.77 (s, 1H), 6.79 (s, 1H), 6.82 (br s, 1H), 7.04 (t, J=7.8Hz, 1H), 7.12 (t, J=7.8Hz, 1H), 7.16-7.23 (m, 3H), 7.30 (d, J=7.8Hz, 1H), 7.33 (d, J=8.4Hz, 2H), 7.56 (d, J=7.8Hz, 1H)
IR (KBr) 3350, 3150, 2950, 1480, 1340, 800, 750, 710 cm⁻¹
MS m/z 286 (M⁺)

### Test Example 1

Porcine ovaries were washed with PBS buffer, and Porcine ovarian granulosa cells (POGC) were collected by aspiration from follicles with a syringe. Cell fraction was recovered as a precipitate by centrifugation of the suspension. The operation of suspending the cell fraction into PBS buffer and subjecting the resulting suspension to centrifugation was repeated three times to wash the cells. The POGC obtained as a precipitate were suspended again into a culture medium (DMEM containing 10% fetal bovine serum) and cell clumps were disrupted by pipetting. The cell suspension was passed through a mesh to remove contaminating tissue fragments, and then pipetted in 24-well plates for cell culture. The cell suspension was cultured for 2 days in a CO₂ incubator according to a conventional procedure (37°C, 5% CO₂). Then, the medium was refreshed to remove floating cells and the cultivation was continued further 2 to 3 days until the cells reached subconfluency (0.7 to 2×10⁵ cell/well). After the attaching cells were washed with serum-free medium, they were cultured in a serum-free DMEM (containing 5 µg/mL of transferrin, and 40 ng/mL of hydrocortisone, 4 mg/mL of bovine serum albumin (BSA), 100nM androstendione) and it was possible to continue to culture cells with relatively immature differentiation. Addition of SNP (sodium nitroprusside, Na₂[Fe(CN)₅NO], 0.5 mM) known as an NO generating reagent to POGC cultured by the above method resulted in death of all the cells which was found on an observation after 6 hours under a light microscopy and an electron microscopy. The cell death was also confirmed by MTT assay. Then, various concentrations of the test compounds were added to the present culture system and the cells were observed after 18 hours. The results are shown in Table 1 where a minimum effective concentration of each compound for more than 95% of inhibition of cell death is described.

**Table 1**

| Inhibiting Effects on Apoptosis of Porcine Ovarian Granulosa Cells by SNP Stimulation | | | |
|---|---|---|---|
| Compound | MEC (µM) | Compound | MEC (µM) |
| 1 | 0.3 | 7 | >10 |
| 2 | 0.3 | 8 | 10 |
| 3 | 10 | 9 | 10 |
| 4 | 10 | 10 | 10 |
| 5 | 10 | 11 | 10 |
| 6 | 10 | | |
| MEC : Minimum effective concentration | | | |

### Test Example 2

According to a known method (*A Dissection and Tissue Culture Manual of the Nervous System,* p. 211, 1989, Alan R, Liss, Inc.), cerebellar granule cells were isolated from the cerebellum of 7 days old rats and cultured. Namely, after conducting isolation procedures described in the above literature, the resulting cells were resuspended in DMEM containing 10% fetal bovine serum, 25 mM KCl and 4 mM glutamine, and seeded in poly-lysine coated plates. After the cells were cultured in a CO₂ incubator for 48 hours according to a conventional method, cytosine-1-β-D(+)-arabinofuranoside (Ara-C) (10 µM) was added thereto, and the cell was continued to culture. The following experiments were conducted with the cells of 7 to 14 days old from the start day of the cultivation, the cells having completed neurite extension sufficiently. Addition of SNP (500 µM) to the cerebellar granule cells cultured as above resulted in death of all the cells which was found on an observation after 14 hours under a light microscopy. Then, various concentrations of the test compounds were added to the present culture system before the addition of SNP and the cells were observed after 14 hours. The results are shown in Table 2 where a minimum effective concentration of each compound for more than 95% of inhibition of cell death is described.

**Table 2**

| Inhibiting Effects on Apoptosis of Cerebellar Granule Cells by SNP Stimulation | | | |
|---|---|---|---|
| Compound | MEC (µM) | Compound | MEC (µM) |
| 1 | 0.3 | 7 | >10 |
| 2 | 0.3 | 8 | 3 |
| 3 | 10 | 9 | 3 |
| 4 | 10 | 10 | 3 |
| 5 | 10 | 11 | 3 |
| 6 | 10 | | |
| MEC : Minimum effective concentration | | | |

### Test Example 3

When antimycin A (1 µM), known as an inhibitor of respiration, was added to cerebellar granule cells cultured as described in Test Example 2, occurrence of cell death was observed under a light microscopy, and all cells were found to be dead on an observation after 2 hours. When the test compound 1 (10 µM) or the test compound 9 (10 µM) coexisted prior to the antimycin A addition, more than 95% of cells were alive on an observation made similarly after 2 hours.

The above results demonstrated that the compounds according to the present invention are able to inhibit cell death in response to a variety of stimuli in various cell types.

### Industrial Applicability

Since the indolylpyrrole derivatives according to the present invention inhibit cell death caused by a wide variety of cell death-inducing stimuli, they are considered to be useful for prevention or treatment of all the diseases whose onset and exacerbation are associated with cell death. Accordingly, the derivatives have uses as remedies for neurodegenerative diseases such as Alzheimer's disease, spinal muscular atrophy, amyotrophic lateral screrosis, Parkinson's disease, Huntington's disease, pigmentary degeneration of the retina, glaucoma, cerebellar degeneration and neonatal jaundice; myasthenia gravis; brain ischemia from apoplexy and the like, and successive delayed neuronal death, ischemic heart disease due to myocardial infarction (myocardial ischemia and disorder after reperfusion); viral myocarditis; autoimmune myocarditis (congestive cardiomyopathy and chronic myocarditis); myocardial disorders or death due to hypertrophic heart and heart failure; arrythmogenic right ventricular cardiomyopathy; alcoholic hepatitis and viral hepatitis; renal diseases such as glomerulonephritis, hemolytic uremic syndrome and the like; acquired immunodeficiency syndrome (AIDS); inflammatory skin disorders such as toxic epidermal necrolysis (TEN) and multiform exudative erythema; alopecia; graft versus host disease (GVH); radiation disorders; disorders due to toxic agents including side effects due to anti-cancer drugs, anti-viral drugs and the like; sepsis; osteomyelo-dysplasia such as aplastic anemia and the like; insulin dependent diabetes; prion diseases such as Creutzfeldt-Jakob's disease and the like; and functional deficiency of transplanted organs and the like, or uses as drugs for stopping or inhibiting progress and exacerbation of the diseases; and also uses as preservatives for organs, tissues and cells.

## Claims

1. A indolylpyrrole derivative represented by the following formula (I): 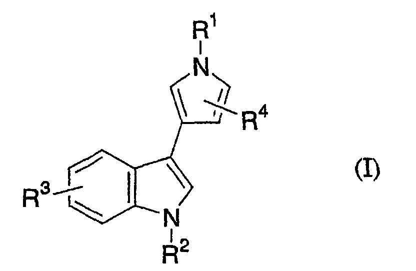 wherein R¹ and R² each independently represents hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or aryloxycarbonyl group which may possess substituent(s), an alkyl- or arylthiocarbonyl group which may possess substituent(s), an aminocarbonyl group which may possess substituent(s), an aminocarbonyloxy group which may possess substituent(s), an alkyl- or arylsulfonyl group which may possess substituent(s), an alkoxyl group which may possess substituent(s), an aryloxy group which may possess substituent(s), or hydroxyl group; R³ represents substituent(s) on an indole ring (at 2-, 4-, 5-, 6-, 7- or more than one position(s) as position number of the indole ring, in the last case substituents may be the same or different), hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or aryloxycarbonyl group which may possess substituent(s), an alkoxy- or aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group which may possess substituent(s), an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group, oxysulfonyl group, cyano group, nitro group, an amino group which may possess substituent(s), or a halogen atom; R¹ and R³, R¹ and R⁴, R² and R⁴, R³ and R⁴, R² and R³, two R³, or two R⁴ (but except the benzene ring fused at 4,5-positions of the pyrrole ring) may be combined to form a hydrocarbon chain or a hydrocarbon chain containing heteroatom(s) which may possess substituent(s); R⁴ represents substituent(s) on a pyrrole ring (at 2-, 4-, 5-, or more than one position(s) as position numbering of pyrrole ring, in the last case substituents may be the same or different), hydrogen atom (but except the case of R¹=R³=H, R²=CH₂Ph), an alkyl group which may possess substituent(s), an alkenyl group which may possess substituent(s), an alkynyl group which may possess substituent(s), an aryl group which may possess substituent(s) (but except the 3-indolyl group substituted at 4-position which may possess substituent(s)), an acyl group which may possess substituent(s) (but except the benzoyl group at 4-position in case of R¹=R³=H, R²=Et, and the 4-methylbenzoyl group at 4-position in case of R¹=1H-imidazol-4-ylmethyl, R²=Me, R³=H) an acyloxy group which may possess substituent(s), an alkyl- or aryloxycarbonyl group which may possess substituent(s) (but except the alkoxycarbonyl group at 4-position), an alkyl- or aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group, an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group (but except monosubstituted derivative at 4-position), cyano group, nitro group, an amino group which may possess substituent(s), or a halogen atom.

2. A cell death inhibitor comprising, as an active ingredient, an indolylpyrrole derivative represented by the following formula (II): 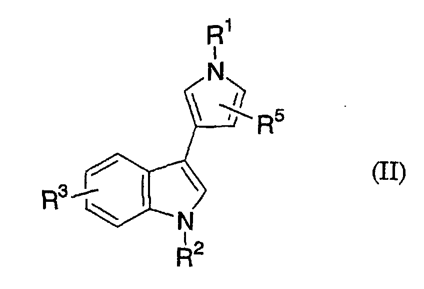 wherein R¹ and R² each independently represents hydrogen atom, an alkyl group which may possess substituent(s), an alkenyl group which may possess substituent(s), an alkynyl group, which may possess substituent(s), an aryl group which may possess substituent(s), an acyl group which may possess substituent(s), an acyloxy group which may possess substituent(s), an alkoxy- or aryloxycarbonyl group which may possess substituent(s), an alkyl- or arylthiocarbonyl group which may possess substituent(s), an aminocarbonyl group which may possess substituent(s), an aminocarbonyloxy group which may possess substituent(s), an alkyl- or arylsulfonyl group which may possess substituent(s), an alkoxyl group which may possess substituent(s), an aryloxy group which may possess substituent(s), or hydroxyl group; R³ represents substituent(s) on an indole ring, and represents, number and position (at 2-, 4-, 5-, 6-, 7- or more than one position(s) as position number of the indole ring, in the last case substituents may be the same or different), hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or aryloxycarbonyl group which may possess substituent(s), an alkoxy- or aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group which may possess substituent(s), an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group, oxysulfonyl group, cyano group, nitro group, an amino group which may possess substituent(s), or a halogen atom; R¹ and R³, R¹ and R⁵, R² and R⁵, R³ and R⁵, R² and R³, two R³, or two R⁵ may be combined to form a hydrocarbon chain or a hydrocarbon chain containing heteroatom(s) which may possess substituent(s); R⁵ represents substituent(s) on a pyrrole ring (at 2-, 4-, 5-, or more than one position(s) as position numbering of pyrrole ring, in the last case substituents may be the same or different), hydrogen atom, an alkyl group which may possess substituent(s), an alkenyl group which may possess substituent(s), an alkynyl group which may possess substituent(s), an aryl group which may possess substituent(s), an acyl group which may possess substituent(s), an acyloxy group which may possess substituent(s), an alkoxy- or an aryloxycarbonyl group which may possess substituent(s), an alkoxy- or an aryloxycarbonyloxy group which may possess substituent(s), an alkyl- or arylthiocarbonyl group which may possess substituent(s), an alkyl- or arylthiocarbonyloxy group which may possess substituent(s), an aminocarbonyl group which may possess substituent(s), an aminocarbonyloxy group which may possess substituent(s), an alkyl- or arylsulfonyl group, an alkyl- or arylsulfinyl group which may possess substituent(s), an alkoxyl group which may possess substituent(s), an aryloxy group which may possess substituent(s), an alkyl- or arylthio group which may possess substituent(s), hydroxyl group, carboxyl group, cyano group, nitro group, an amino group which may possess substituent(s), or a halogen atom,
or a pharmaceutically acceptable salt thereof.

3. A drug for treating or preventing progress of symptoms, through inhibiting death of neurons, of neurodegenerative diseases such as Alzheimer's disease, spinal muscular, amyotrophic lateral screrosis, Parkinson's disease, Huntington's disease, pigmentary degeneration of the retina, glaucoma and cerebellar degeneration, comprising an indolylpyrrole derivative represented by the above formula (II) or a pharmaceutically acceptable salt thereof as an active ingredient.

4. A drug for treating or preventing progress of symptoms, through inhibiting death of neurons, of neonatal jaundice, comprising an indolylpyrrole derivative represented by the above formula (II) or a pharmaceutically acceptable salt thereof as an active ingredient.

5. A drug for treating or preventing progress of symptoms, through inhibiting cell death, of myasthenia gravis, comprising an indolylpyrrole derivative represented by the above formula (II) or a pharmaceutically acceptable salt thereof as an active ingredient.

6. A drug for treating or preventing progress of symptoms, through inhibiting death of neurons, of brain ischemia and delayed neuronal death (DND), comprising an indolylpyrrole derivative represented by the above formula (II) or a pharmaceutically acceptable salt thereof as an active ingredient.

7. A drug for treating or preventing progress of symptoms, through inhibiting death of myocardial cells, of ischemic heart disease, viral myocarditis, autoimmune myocarditis, myocardial disorders/cell death due to hypertrophic heart and heart failure, or arrythmogenic right ventricular cardiomyopathy, comprising an indolylpyrrole derivative represented by the above formula (II) or a pharmaceutically acceptable salt thereof as an active ingredient.

8. A drug for treating or preventing progress of symptoms, through inhibiting death of hepatic cells, of alcoholic hepatitis or viral hepatitis, comprising an indolylpyrrole derivative represented by the above formula (II) or a pharmaceutically acceptable salt thereof as an active ingredient.

9. A drug for treating or preventing progress of symptoms, through inhibiting death of renal cells, of renal diseases, comprising an indolylpyrrole derivative represented by the above formula (II) or a pharmaceutically acceptable salt thereof as an active ingredient.

10. A drug for treating or preventing progress of symptoms, through inhibiting excessive death of T-cells, of acquired immunodeficiency syndrome (AIDS), comprising an indolylpyrrole derivative represented by the above formula (II) or a pharmaceutically acceptable salt thereof as an active ingredient.

11. A drug for treating or preventing progress of symptoms, through inhibiting cell death, of inflammatory skin disorders, alopecia, or graft versus host disease (GVH), comprising an indolylpyrrole derivative represented by the above formula (II) or a pharmaceutically acceptable salt thereof as an active ingredient.

12. A drug for treating or preventing disorders or side effects, through inhibiting cell death, of disorders due to radiation or drugs, comprising an indolylpyrrole derivative represented by the above formula (II) or a pharmaceutically acceptable salt thereof as an active ingredient.

13. A drug for treating or preventing progress of symptoms, through inhibiting cell death, of sepsis, comprising an indolylpyrrole derivative represented by the above formula (II) or a pharmaceutically acceptable salt thereof as an active ingredient.

14. A drug for treating or preventing progress of symptoms, through inhibiting death of cells derived from bone marrow, of osteomyelo-dysplasia, comprising an indolylpyrrole derivative represented by the above formula (II) or a pharmaceutically acceptable salt thereof as an active ingredient.

15. A drug for treating or preventing progress of symptoms, through inhibiting cell death, of insulin dependent diabetes, comprising an indolylpyrrole derivative represented by the above formula (II) or a pharmaceutically acceptable salt thereof as an active ingredient.

16. A drug for treating or preventing progress of symptoms, through inhibiting death of neurons, of prion diseases, comprising an indolylpyrrole derivative represented by the above formula (II) or a pharmaceutically acceptable salt thereof as an active ingredient.

17. A drug for treating or preventing functional deficiency of transplanted organs, tissues or cells at transplantation of organs, tissues or cells, comprising an indolylpyrrole derivative represented by the above formula (II) or a pharmaceutically acceptable salt thereof as an active ingredient.

18. A preservative for organs, tissues or cells, comprising an indolylpyrrole derivative represented by the above formula (II) or a pharmaceutically acceptable salt thereof as an active ingredient.

19. A medicament, comprising an indolylpyrrole derivative represented by the above formula (II) or a pharmaceutically acceptable salt thereof as an active ingredient.
